# EUROPEAN PATENT APPLICATION

(11) **EP 4 480 388 A1**
(43) Date of publication of application: **25.12.2024**
(21) Application number: 23756367.1
(22) Date of filing: 14.02.2023
(51) Int. Cl.: A61B 1/24, A61B 1/00, A61B 1/045, A61B 1/06, A61C 19/00, A61C 19/04

(54) **INTRAORAL CAMERA, ILLUMINATION CONTROL DEVICE, AND ILLUMINATION CONTROL METHOD**

(30) Priority: 17.02.2022 JP 2022022756
(71) Applicant: Panasonic Intellectual Property Management Co., Ltd., Kadoma-shi, Osaka 571-0057 (JP)
(72) Inventor: OHTSUKA, Yoshio, Kadoma-shi, Osaka 571-0057 (JP); HAMASAKI, Takeshi, Kadoma-shi, Osaka 571-0057 (JP)
(74) Representative: Novagraaf International SA
(86) International application number: PCT/JP2023/005005
(87) International publication number: WO 2023/157842

(57) **Abstract**

An intraoral camera (11) includes an imaging unit (21), an illuminator (23), and an illumination controller (34). The imaging unit (21) generates image data by capturing an image of a tooth inside the mouth of a user. The illuminator (23) illuminates the tooth. The illumination controller (34) controls at least one of the illumination intensity or the color temperature of the illuminator (23) to bring the color temperature of reflected light from the tooth that is based on the image data, closer to a target color temperature based on the second color temperature of the illuminator (23).

## Description

### [Technical Field]

The present disclosure relates to an intraoral camera, an illumination control device, and an illumination control method.

### [Background Art]

Patent Literature (PTL) 1 discloses an intraoral observation device (an intraoral camera) including a light source (an illuminator) for illuminating the interior of a mouth and a light receiver (an imaging unit) for receiving reflected light from inside the mouth after light is emitted from the light source and reflected off the interior of the mouth.

### [Citation List]

### [Patent Literature]

[PTL 1] Japanese Unexamined Patent Application Publication No. 2008-86554

### [Summary of Invention]

### [Technical Problem]

Incidentally, it is expected for an intraoral camera for capturing an image of the interior of a mouth to accurately obtain a teeth condition such as formation of dental plaque.

Thus, the present disclosure provides an intraoral camera, an illumination control device, and an illumination control method that can accurately obtain a teeth condition.

### [Solution to Problem]

An intraoral camera according to one aspect of the present disclosure includes: an imaging unit that generates image data by capturing an image of a tooth inside the mouth of a user; an illuminator that illuminates the tooth; and an illumination controller that controls at least one of the illumination intensity or the color temperature of the illuminator to bring the first color temperature of reflected light from the tooth closer to a target color temperature based on the second color temperature of the illuminator, the first color temperature being based on the image data.

An illumination control device according to another aspect of the present disclosure controls the illuminator of an intraoral camera including an imaging unit that generates image data by capturing an image of a tooth inside the mouth of a user and the illuminator that illuminates the tooth. The illumination control device includes: an obtainer that obtains the image data generated by the imaging unit; and an illumination controller that controls at least one of the illumination intensity or the color temperature of the illuminator to bring the first color temperature of reflected light from the tooth closer to a target color temperature based on the second color temperature of the illuminator, the first color temperature being based on the image data.

An illumination control method according to still another aspect of the present disclosure includes: generating image data by capturing an image of a tooth inside the mouth of a user; illuminating the tooth; and controlling at least one of the illumination intensity or the color temperature of an illuminator to bring the first color temperature of reflected light from the tooth closer to a target color temperature based on the second color temperature of the illuminator, the first color temperature being based on the image data.

### [Advantageous Effects of Invention]

The present disclosure can provide an intraoral camera, an illumination control device, and an illumination control method that can accurately obtain a teeth condition.

### [Brief Description of Drawings]

[FIG. 1]

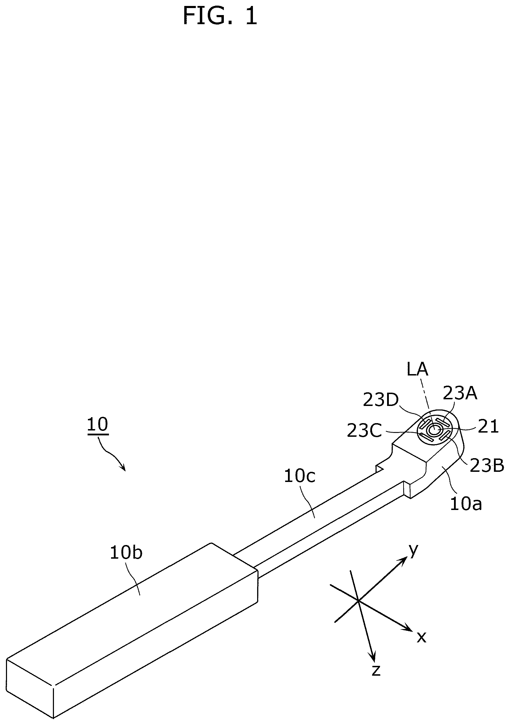

   FIG. 1 is a perspective view of the intraoral camera of an intraoral camera system according to Embodiment 1.
[FIG. 2]

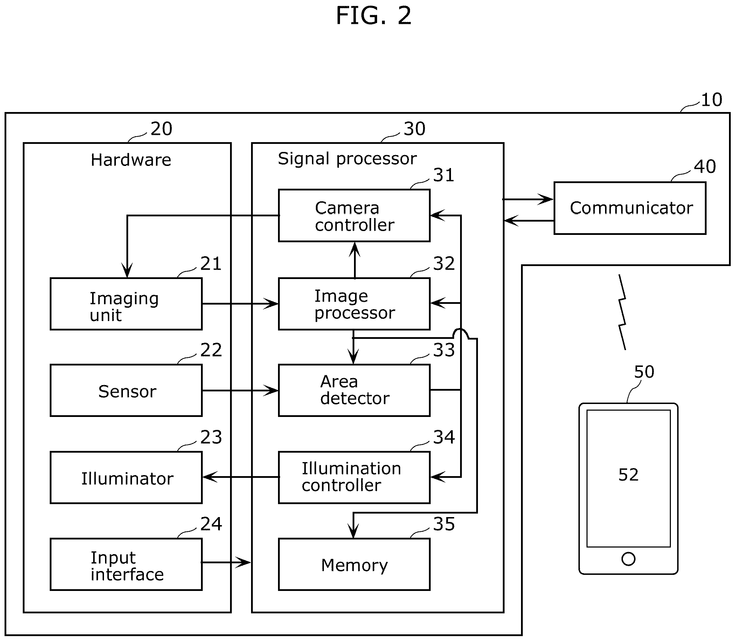

   FIG. 2 illustrates a schematic configuration of the intraoral camera system according to Embodiment 1.
[FIG. 3]

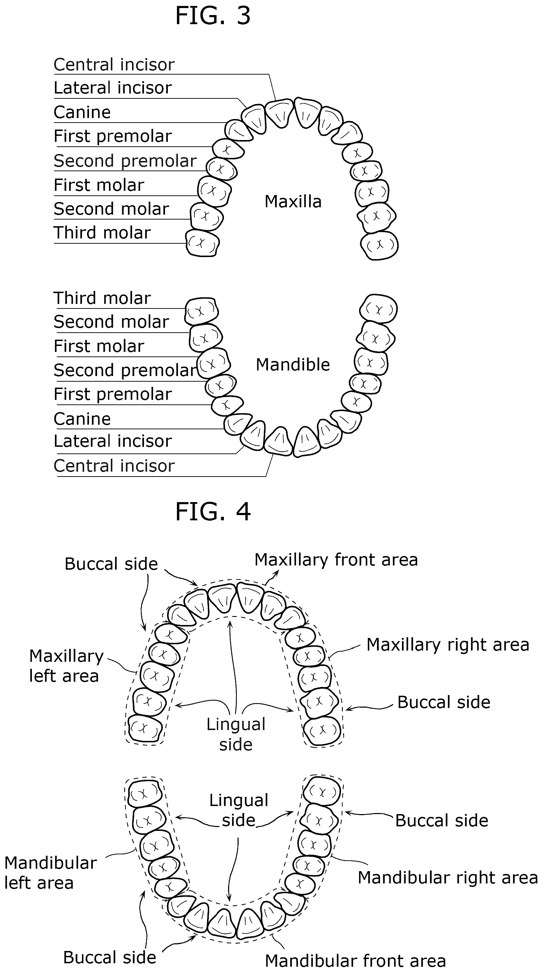

   FIG. 3 illustrates teeth inside a mouth according to Embodiment 1.
[FIG. 4]
   FIG. 4 illustrates an example of intraoral areas according to Embodiment 1.
[FIG. 5]

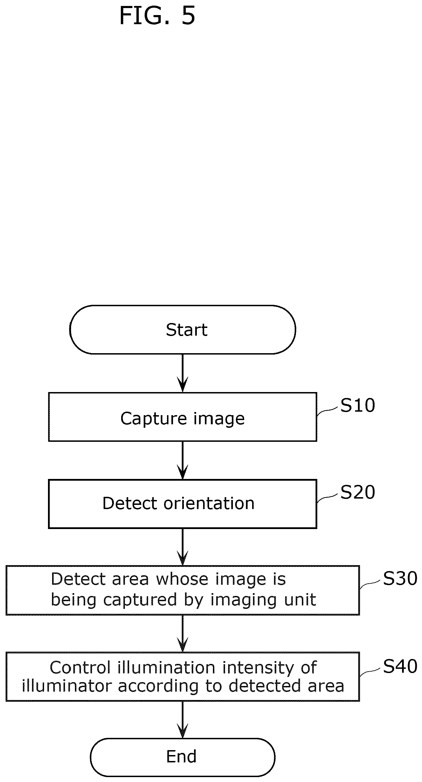

   FIG. 5 is a flowchart illustrating an operation of the intraoral camera according to Embodiment 1.
[FIG. 6]

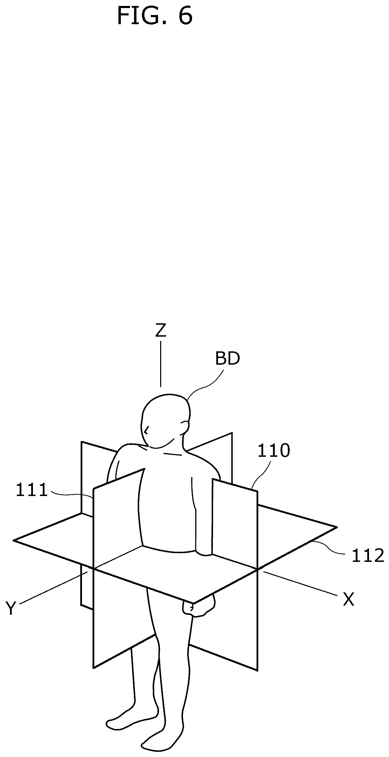

   FIG. 6 illustrates relationships between projective planes and a standing user, according to a variation of Embodiment 1.
[FIG. 7]

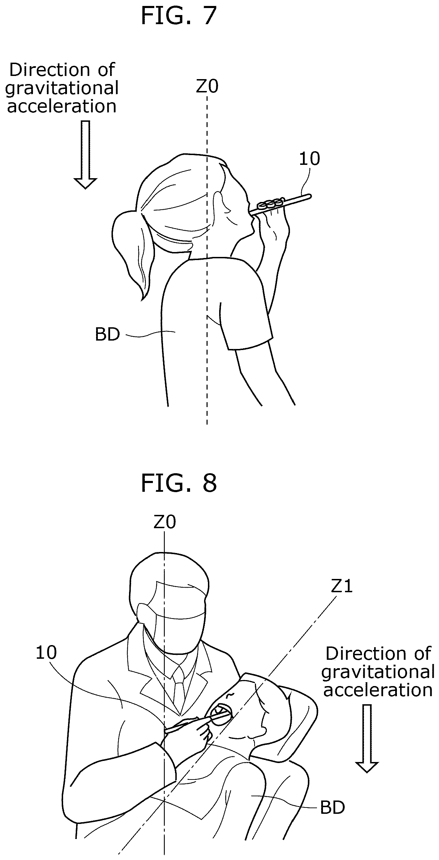

   FIG. 7 illustrates an example of the posture of a user during use of an intraoral camera, according to the variation of Embodiment 1.
[FIG. 8]
   FIG. 8 illustrates an example of the posture of a user during use of the intraoral camera, according to the variation of Embodiment 1.
[FIG. 9]

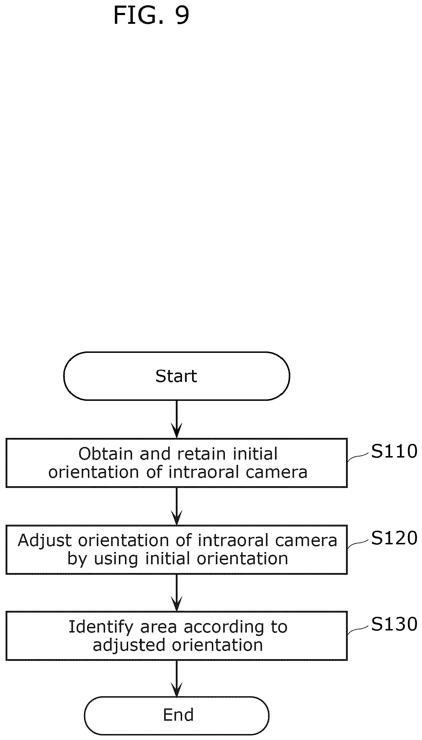

   FIG. 9 is a flowchart illustrating image processing according to the variation of Embodiment 1.
[FIG. 10]

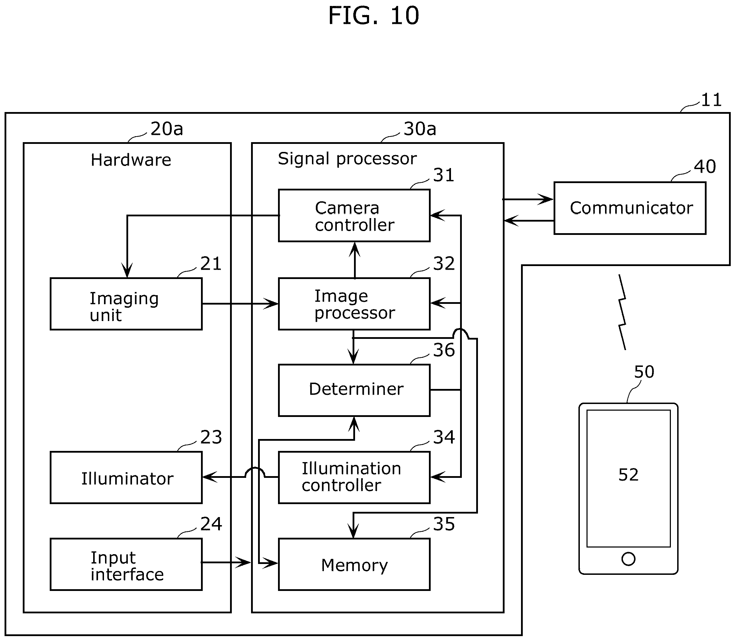

   FIG. 10 illustrates a schematic configuration of an intraoral camera system according to Embodiment 2.
[FIG. 11]

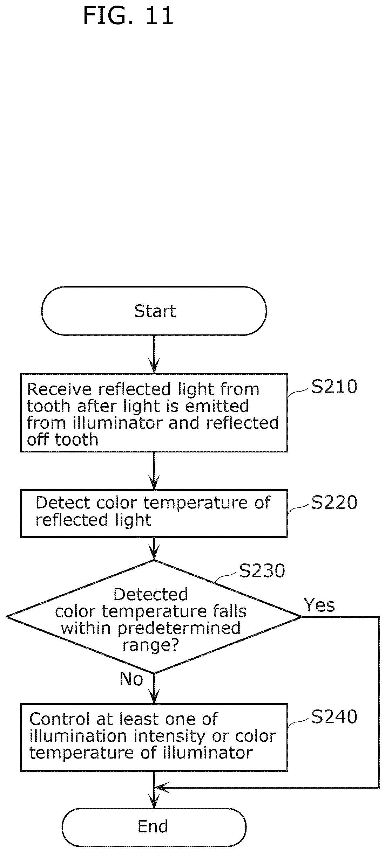

   FIG. 11 is a flowchart illustrating an operation of an intraoral camera according to Embodiment 2.

### [Description of Embodiments]

An intraoral camera according to the first aspect of the present disclosure includes: an imaging unit that generates image data by capturing an image of a tooth inside the mouth of a user; an illuminator that illuminates the tooth; and an illumination controller that controls at least one of the illumination intensity or the color temperature of the illuminator to bring the first color temperature of reflected light from the tooth closer to a target color temperature based on the second color temperature of the illuminator, the first color temperature being based on the image data.

Thus, it is possible to capture the image data having a color temperature close to the target color temperature regardless of the position of the tooth, which can suppress variations in the color temperature of the image data due to the effects of, for example, external light. For example, reducing the effects of the external light by color temperature control makes it easier for the intraoral camera to substantially fix the value of white balance gain suitable for the illuminator (e.g., the light-emitting devices of the illuminator), which enables detection of dental plaque with less variations due to the environment. Thus, the intraoral camera according to one aspect of the present disclosure can accurately obtain a teeth condition even if there are the effects of the external light, for example.

In addition, an intraoral camera according to the second aspect of the present disclosure is the intraoral camera according to the first aspect. For instance, the illumination controller may control the at least one of the illumination intensity or the color temperature of the illuminator to bring the first color temperature within a predetermined range including the target color temperature.

Since the illuminator is controlled so that the color temperature of the reflected light is brought within the predetermined range regardless of the position of the tooth, the intraoral camera can obtain the teeth condition more accurately.

In addition, an intraoral camera according to the third aspect of the present disclosure is the intraoral camera according to the first or second aspect, and may further include, for instance, a determiner that identifies, from first image data captured by the imaging unit, the position of the tooth whose image is being captured by the imaging unit. The illumination controller may control the at least one of the illumination intensity or the color temperature of the illuminator by further using the result of the identification by the determiner.

Since the at least one of the illumination intensity or the color temperature of the illuminator is controlled according to the position of the tooth, the intraoral camera can obtain the teeth condition more accurately.

In addition, an intraoral camera according to the fourth aspect of the present disclosure is the intraoral camera according to the third aspect. For instance, the determiner may identify the position of the tooth whose image is being captured by the imaging unit from the first image data and second image data that indicates typical shapes of teeth.

This makes it possible to readily identify the position of the tooth by using the second image indicating the typical shapes of the teeth.

In addition, an intraoral camera according to the fifth aspect of the present disclosure is the intraoral camera according to the third aspect, and may further include, for example, a storage storing third image data that is pre-captured intraoral image data and includes the dentition of the user of the intraoral camera. The determiner may identify, from the first image data and the third image data, the position of the tooth whose image is being captured by the imaging unit.

This makes it possible to identify the position of the tooth from the image data of the teeth of the user, which improves the accuracy of identifying the position of the tooth.

In addition, an intraoral camera according to the sixth aspect of the present disclosure is the intraoral camera according to the third aspect, and may further include, for example, a communicator that transmits, to the user of the intraoral camera, notification information notifying an area whose image is to be captured by the imaging unit among a plurality of areas inside the mouth which are defined by dividing the dentition of the user into sections. The determiner may determine that an image captured by the imaging unit after the user is notified of the area indicated by the notification information is an image of the area.

Thus, it is possible to omit the processing for identifying the position of the tooth whose image is being captured, which can lead to reduction in the amount of processing in the intraoral camera.

In addition, an intraoral camera according to the seventh aspect of the present disclosure is the intraoral camera according to one of the first to sixth aspects. For instance, the target color temperature may be set according to the color temperature of a tooth in an image captured first after the intraoral camera is put inside the mouth, and the illumination controller may control the at least one of the illumination intensity or the color temperature of the illuminator to bring the first color temperature across the entire intraoral area closer to the target color temperature set.

This makes it possible to bring the color temperature of another tooth closer to the color temperature of the tooth in the image captured first.

In addition, an intraoral camera according to the eighth aspect of the present disclosure is the intraoral camera according to one of the first to the sixth aspects. For instance, the illumination controller may control the at least one of the illumination intensity or the color temperature of the illuminator according to the color temperature of a glossy area strongly affected by light emitted from the illuminator among a plurality of areas inside the mouth which are defined by dividing a dentition of the user into sections.

This makes it possible to bring the color temperature of another area closer to the color temperature of the glossy area.

In addition, an intraoral camera according to the nineth aspect of the present disclosure is an illumination control device that controls the illuminator of an intraoral camera including an imaging unit that generates image data by capturing an image of a tooth inside the mouth of a user and the illuminator that illuminates the tooth. The illumination control device includes: an obtainer that obtains the image data generated by the imaging unit; and an illumination controller that controls at least one of the illumination intensity or the color temperature of the illuminator to bring the first color temperature of reflected light from the tooth closer to a target color temperature based on the second color temperature of the illuminator, the first color temperature being based on the image data. In addition, an illumination control method according to the 10th aspect of the present disclosure includes generating image data by capturing an image of a tooth inside the mouth of a user; illuminating the tooth; and controlling at least one of the illumination intensity or the color temperature of an illuminator to bring the first color temperature of reflected light from the tooth closer to a target color temperature based on the second color temperature of the illuminator, the first color temperature being based on the image data.

In this way, effects similar to those of the intraoral camera described above can be obtained.

In addition, an intraoral camera according to the 11th aspect of the present disclosure may include, for instance, an imaging unit that generates image data by capturing an image of a tooth inside the mouth of a user, an area detector that detects an area whose image is being captured by the imaging unit among a plurality of areas inside the mouth which are defined by dividing the dentition of the user into sections, an illuminator that illuminates the area detected, and an illumination controller that controls the illumination intensity of the illuminator according to the area detected.

Since the illuminator illuminates the area at an illumination intensity suitable for the area whose image is being captured by the imaging unit, the intraoral camera can properly control the illumination intensity of the illuminator.

In addition, an intraoral camera according to the 12th aspect of the present disclosure is the intraoral camera according to the 11th aspect. For instance, the plurality of areas may include a first area including an anterior tooth and a second area positioned more posterior (towards the back) than the first area. The illumination controller may change the illumination intensity of the illuminator between the first area and the second area.

Since the intraoral camera changes the illumination intensity of the illuminator between the first area and the second area, it is possible to control the illumination intensity of the illuminator more properly.

In addition, an intraoral camera according to the 13th aspect of the present disclosure is the intraoral camera according to the 11th or 12th aspect. For instance, the illumination controller may control the illuminator to cause an illumination intensity for the second area to be higher than an illumination intensity for the first area.

Thus, when the first area is illuminated with the external light, it is possible to bring the brightness of the image of the first area and the brightness of the image of the second area closer to each other. It should be noted that the external light is light different from light emitted by the illuminator, and examples of the external light include the light of lighting equipment and the sunlight.

In addition, an intraoral camera according to the 14th aspect of the present disclosure is the intraoral camera according to one of the 11th to 13th aspects. For instance, the area detector may further detect from which of the buccal side or the lingual side inside the mouth the imaging unit is capturing the image of the area. The illumination controller may change the illumination intensity of the illuminator between when the imaging unit is capturing the image of the area from the buccal side and when the imaging unit is capturing the image of the area from the lingual side.

Since the intraoral camera changes the illumination intensity of the illuminator, depending on from which of the buccal side or the lingual side the imaging unit is capturing the image (that is, according to the imaging direction of the imaging unit), it is possible to control the illumination intensity of the illuminator more properly.

In addition, an intraoral camera according to the 15th aspect of the present disclosure is the intraoral camera according to the 13th aspect. For instance, the area detector may further detect from which of the buccal side or the lingual side inside the mouth the imaging unit is capturing the image of the area. The illumination controller may perform first control and second control which may be different from one another, the first control being control for the illuminator performed according to whether an image of the first area is being captured from the lingual side or from the buccal side, the second control being control for the illuminator performed according to whether an image of the second area is being captured from the lingual side or from the buccal side.

Since the intraoral camera changes the control details of the illumination intensity of the illuminator between the first area and the second area, it is possible to control the illumination intensity of the illuminator more properly.

In addition, an intraoral camera according to the 16th aspect of the present disclosure is the intraoral camera according to the 15th aspect. For instance, in the first control, when the image of the first area is being captured from the front of the user, the illumination controller may control the illuminator to illuminate at a higher illumination intensity than when the image of the first area is being captured from the buccal side. In the second control, when the image of the second area is being captured from the lingual side, the illumination controller may control the illuminator to illuminate at a lower illumination intensity than when the area of the second area is being captured from the buccal side.

This enables the intraoral camera to bring, closer to each other, the brightness of the image of the first area captured from the front from the user (an image susceptible to the external light) and the brightness of the image of the first area captured from the lingual side (an image less susceptible to the external light). In addition, the intraoral camera can bring, closer to each other, the brightness of the image of the second area captured from the lingual side (an image susceptible to the external light) and the brightness of the image of the second area captured from the buccal side (an image less susceptible to the external light). Thus, the intraoral camera can properly control the illumination intensity of the illuminator so that it is possible to suppress generation of image data in which the brightness differs depending on the area due to differences in the effects of the external light.

In addition, an intraoral camera according to the 17th aspect of the present disclosure is the intraoral camera according to one of the 11th to 16th aspects. For instance, the plurality of areas may include two or more areas included in a maximally area and two or more areas included in a mandibular area.

This enables the intraoral camera to control the illumination intensity more minutely.

In addition, an intraoral camera according to the 18th aspect of the present disclosure is the intraoral camera according to one of the 11th to 17th aspects. For instance, the intraoral camera may include, for example, an orientation detector that detects the orientation of the imaging unit from the output of a multi-axis acceleration sensor. The area detector may detect, from the orientation detected, the area whose image is being captured by the imaging unit.

This enables the intraoral camera to detect, from the orientation of the imaging unit, the area whose image is being/is to be captured.

In addition, an intraoral camera according to the 19th aspect of the present disclosure is the intraoral camera according to one of the 11th to 18th aspects. For instance, the area detector may identify the type of the tooth in the image data and detect, from the type of the tooth identified, the area whose image is being captured by the imaging unit.

This enables the intraoral camera to detect the area whose image is being/is to be captured, by using the image data captured by the imaging unit. That is, the intraoral camera need not include a dedicated element for detecting the area whose image is being/is to be captured. Thus, the intraoral camera can properly control the illumination intensity of the illuminator with a simple configuration.

In addition, an intraoral camera according to the 20th aspect of the present disclosure is the intraoral camera according to the 18th aspect. For instance, the initial orientation of the imaging unit which is a predetermined orientation of the imaging unit may be obtained. The area detector may adjust the orientation by using the initial orientation and detect, from the orientation adjusted, the area whose image is being captured by the imaging unit among the plurality of areas.

Thus, by adjusting the orientation of the imaging unit according to the posture of the user, the intraoral camera can properly control the illumination intensity of the illuminator regardless of the posture of the user.

In addition, an illumination control method according to the 21st aspect of the present disclosure includes generating image data by capturing an image of a tooth inside the mouth of a user, detecting an area whose image is being captured by the imaging unit among a plurality of areas inside the mouth which are defined by dividing a dentition into sections, and controlling, according to the area detected, the illumination intensity of the illuminator that illuminates the area detected.

Thus, the illumination control method provides effects similar to those of the intraoral camera system described above.

It should be noted that these general or specific aspects may be embodied as a system, a method, an integrated circuit, a computer program, or a computer-readable recording medium such as a CD-ROM or may be embodied as any combination of the system, the method, the integrated circuit, the computer program, and the recording medium.

It should be noted that the embodiments described below each indicate a general or specific example. The numerical values, shapes, structural elements, arrangements and connections of the structural elements, steps, order of steps, and other details indicated in the embodiments described below are examples, and therefore do not intend to limit the present disclosure. In addition, among the structural elements described in the embodiments below, those not recited in the independent claims are described as optional structural elements.

In addition, the drawings are schematic drawings and are not necessarily precisely drawn. Accordingly, the scales used in the drawings are not necessarily the same, for example. In addition, in the drawings, substantially the same elements are assigned the same reference signs, and overlapping explanations are omitted or simplified.

In addition, in the specification, terms describing a relationship between elements, such as parallel, identical (match), and orthogonal, terms describing the shapes of elements such as a ring shape, numerical values, and numerical value ranges are not limited to having precise meanings but include substantially equivalent ranges that encompass, for example, a difference of around several percent (or around 10%).

### [Embodiment 1]

An intraoral camera and an illumination control method according to Embodiment 1 are described below with reference to FIGS. 1 to 5.

### [1-1. Configuration of Intraoral Camera System]

A configuration of an intraoral camera system including the intraoral camera according to Embodiment 1 is described below with reference to FIGS. 1 and 2. FIG. 1 is a perspective view of the intraoral camera of an intraoral camera system according to Embodiment 1.

As illustrated in FIG. 1, intraoral camera 10 includes a toothbrush-shaped case that can be handled by one hand. The case includes head 10a, handle 10b, and neck 10c. Head 10a is put inside a user's mouth when a dentition image is captured. Handle 10b is designed to be held by a user. Neck 10c connects head 10a to handle 10b.

Imaging unit 21 is incorporated into head 10a and neck 10c. Imaging unit 21 includes an image sensor (not illustrated) and a lens (not illustrated) disposed along optical axis LA of imaging unit 21.

The image sensor is an imaging device, such as a complementary metal oxide semiconductor (CMOS) sensor or a charge coupled device (CCD), and the lens forms an image of a tooth. The image sensor outputs a signal (image data) corresponding to the formed image to an external device.

In addition, intraoral camera 10 is equipped with first to fourth light-emitting diodes (LEDs) 23A to 23D as illuminators (illumination devices) that illuminate a target tooth during image capturing. First to fourth LEDs 23A to 23D are, for example, white LEDs.

FIG. 2 illustrates a schematic configuration of the intraoral camera system according to Embodiment 1.

As illustrated in FIG. 2, in overview, the intraoral camera system according to Embodiment 1 is capable of capturing an image of a dentition in a state in which the illuminator is illuminating the dentition at an illumination intensity suitable for an intraoral area whose image is captured by imaging unit 21. It should be noted that the intraoral area (image capturing area) whose image is captured by imaging unit 21 is an area currently being captured by imaging unit 21 or an area to be captured by imaging unit 21.

As illustrated in FIG. 2, the intraoral camera system includes intraoral camera 10 and portable terminal 50. In the intraoral camera system, when intraoral camera 10 captures an image of each of areas inside the mouth, in order to equalize the brightness of each image captured, irrespective of the degrees of the effects of external light, the illumination intensity of illuminator 23 is controlled according to the intraoral area whose image is being/is to be captured by imaging unit 21. The external light is light different from light emitted by illuminator 23, and examples of the external light include the light of lighting equipment and sunlight.

Portable terminal 50 is, for example, a wirelessly communicable smartphone or tablet terminal. Portable terminal 50 includes, as an input device and an output device, touch screen 52 capable of displaying, for example, a dentition image. Portable terminal 50 functions as the user interface of the intraoral camera system.

Intraoral camera 10 includes hardware 20, signal processor 30, and communicator 40.

Hardware 20 is a physical element of intraoral camera 10 and includes imaging unit 21, sensor 22, illuminator 23, and input interface 24.

Imaging unit 21 generates image data by capturing an image of a tooth inside the user's mouth. Imaging unit 21 receives a control signal from camera controller 31, performs an operation such as image capturing in accordance with the received control signal, and outputs, to image processor 32, image data representing a video or a still image obtained by the image capturing operation. Imaging unit 21 includes the image sensor and the lens described above. Imaging unit 21 is an example of an imaging unit. Although the image data is a dentition image showing teeth, the image data may show at least one tooth.

Sensor 22 is a sensor that performs sensing to detect the orientation of intraoral camera 10. For instance, sensor 22 generates information (sensing data) such as acceleration and/or angular acceleration corresponding to the orientation and/or movement of imaging unit 21, and outputs the generated information to area detector 33. Sensor 22 is, for example, a six-axis sensor including a three-axis acceleration sensor and a three-axis gyro sensor. However, sensor 22 may be a multi-axis (here, x, y, and z-axis, that is, three-axis) acceleration sensor. For instance, as illustrated in FIG. 1, the z-axis is identical to optical axis LA. The y-axis is parallel to an imaging plane and extends in a longitudinal direction of the intraoral camera. The x-axis is parallel to the imaging plane and orthogonal to the y-axis. It should be noted that the orientation of imaging unit 21 is an orientation determined by the imaging direction of imaging unit 21 and is, for example, a three-dimensional orientation of imaging unit 21. The x-axis, the y-axis, and the z-axis represent, for example, three axes in a three-dimensional orthogonal coordinate system.

A piezo-resistive type, capacitive type, or heat detection type micro-electro-mechanical system (MEMS) sensor may be used as sensor 22. Although not illustrated in the figures, sensor 22 may include a correction circuit for correcting, for example, the balance of sensor sensitivity between the axes, the temperature characteristics of sensitivity, and temperature drift. In addition, sensor 22 may include a bandpass filter (a low pass filter) for removing, for example, dynamic acceleration components and noise. In addition, sensor 22 may reduce noise by smoothing a waveform output by the acceleration sensor.

Illuminator 23 illuminates an area whose image is being/is to be captured by imaging unit 21 among a plurality of intraoral areas. For instance, illuminator 23 illuminates the area detected by area detector 33. Illuminator 23 includes first to fourth LEDs 23A to 23D, which are described above. For instance, first to fourth LEDs 23A to 23D illuminate the image capturing area from different directions. This can suppress a shadow from appearing in the image capturing area.

In each of first to fourth LEDs 23A to 23D, at least the brightness of light is controllable. In each of first to fourth LEDs 23A to 23D, the brightness of light and the color of light may be controllable. First to fourth LEDs 23A to 23D surround imaging unit 21.

The illumination intensity (light emission intensity) of illuminator 23 is controlled according to the image capturing area. The illumination intensity of each of first to fourth LEDs 23A to 23D may be controlled to be the same and may be controlled be different. It should be noted that the number of the LEDs of illuminator 23 is not limited to a particular number. Illuminator 23 may include one LED or at least five LEDs. In addition, illuminator 23 is not limited to having LED(s) as the light source and may include another light source.

Input interface 24 accepts input from the user. Input interface 24 is, for example, a push button and may accept input by sound.

In addition, hardware 20 may further include a battery (e.g., a secondary battery) for supplying power to the structural elements of intraoral camera 10, a coil for enabling wireless charging by an external charger connected to a commercial power source, and an actuator necessary to perform at least one of composition adjustment or focus adjustment.

Signal processor 30 includes functional elements embodied as the elements of a central processing unit (CPU) or a microprocessor unit (MPU) which performs various processing tasks described later and memory 35 such as RAM and ROM storing program(s) for causing the functional elements to perform the various processing tasks. Signal processor 30 includes camera controller 31, image processor 32, area detector 33, illumination controller 34, and memory 35.

Camera controller 31 is included in, for example, handle 10b of intraoral camera 10 and controls imaging unit 21. Camera controller 31 controls at least one of the aperture or shutter speed of imaging unit 21 according to, for example, at least one of a control signal from image processor 32 or the result of detection by area detector 33. The result of detection is the result of detection of the image capturing area by imaging unit 21. When for instance the image capturing area is a front area inside the mouth (e.g., the maxillary front area and the mandibular front area illustrated in FIG. 4), camera controller 31 may control imaging unit 21 to reduce the aperture, compared with when the image capturing area is an area different from the front area (e.g., the maxillary left area, the maxillary right area, the mandibular left area, and the mandibular right area illustrated in FIG. 4). In addition, camera controller 31 may control imaging unit 21 so that different aperture setting values are set for the respective six areas: the maxillary left area, the maxillary front area, the maxillary right area, the mandibular left area, the mandibular front area, and the mandibular right area. It should be noted that a table associating each area with the setting values of the aperture and the shutter speed is preset and stored in memory 35.

Image processor 32 is included in, for example, handle 10b of intraoral camera 10. Image processor 32 obtains a dentition image (image data) captured by imaging unit 21 on the basis of the result of detection by area detector 33, performs image processing on the obtained dentition image, and outputs the dentition image which has undergone the image processing to camera controller 31 and area detector 33. In addition, image processor 32 may output the dentition image which has undergone the image processing to memory 35, and store the dentition image which has undergone the image processing in memory 35. Image processor 32 may function as an obtainer that obtains image data from imaging unit 21.

Image processor 32 is, for example, a circuit and performs, on the dentition image, the image processing such as noise removal, automatic white balance (AWB) processing, and edge enhancement processing. The image processing is performed in order to, for example, improve the accuracy of area detector 33. When for instance the dentition image is image data having at least predetermined brightness (a pixel value), image processor 32 may perform the edge enhancement processing after lowering the brightness of the dentition image. The brightness of the dentition image includes, for example, the maximum value, the mean value, and the median value of the pixel value of each pixel. However, the brightness of the dentition image is not limited to the above values.

Image processor 32 may change the details of the image processing according to the result of detection by area detector 33. Image processor 32 may change the details of the image processing, depending on which of the six areas the image capturing area is. Here, the six areas includes the maxillary left area, the maxillary front area, the maxillary right area, the mandibular left area, the mandibular front area, and the mandibular right area.

It should be noted that the dentition image output from image processor 32 (the dentition image which has undergone the image processing) may be transmitted to portable terminal 50 via communicator 40 and displayed on touch screen 52 of portable terminal 50. In this way, it is possible to present the dentition image to the user.

Area detector 33 detects the area whose image is being/is to be captured by imaging unit 21 among the plurality of intraoral areas defined by dividing a dentition into sections. The plurality of areas are described later with reference to FIGS. 3 and 4.

By using at least one of image data from imaging unit 21 or sensing data from sensor 22, area detector 33 detects (identifies) the area whose image is being/is to be captured by imaging unit 21 from among the plurality of areas. Area detector 33 may identify the types of teeth (see FIG. 3) in the image data and detect the area whose image is being/to be captured by imaging unit 21 from the identified types of the teeth. Area detector 33 may identify the types of the teeth in the image data by using a machine learning model trained to output the type of a tooth in image data in response to receiving the image data as input. Area detector 33 may identify the types of the teeth in the image data from the degree of matching with the pre-obtained image data of each tooth. In addition, area detector 33 may identify the types of the teeth in the image data by using a machine learning model trained to output the type of a tooth in image data in response to receiving the image data and sensing data as input. It should be noted that the method of identifying a tooth from image data is not limited to the above method, and any existing method may be used.

In addition, area detector 33 may detect the orientation of imaging unit 21 from the sensing data and detect the area whose image is being/is to be captured by imaging unit 21 from the detected orientation. Area detector 33 may function as an orientation detector for detecting the orientation of imaging unit 21. A method of detecting the orientation of imaging unit 21 from the sensing data is described later.

It should be noted that area detector 33 may detect at least which of a first area or a second area the image capturing area of imaging unit 21 is. Here, the first area includes anterior teeth (e.g., the maxillary front area and the mandibular front area illustrated in FIG. 4). The second area (e.g., the maxillary left area, the maxillary right area, the mandibular left area, and the mandibular right area illustrated in FIG. 4) is positioned more posterior (towards the back) than the first area inside the mouth. The first area(s) and the second area(s) are examples of a plurality of areas inside the mouth defined by dividing a dentition into sections. The anterior teeth include, for example, at least one of a central incisor or a lateral incisor.

Illumination controller 34 is included in, for example, handle 10b of intraoral camera 10 and controls the light emission forms of first to fourth LEDs 23A to 23D according to the result of detection by area detector 33. Illumination controller 34 controls the illumination intensity of illuminator 23 according to the image capturing area detected by area detector 33. Control of the illumination intensity by illumination controller 34 is described later. It should be noted that control of the light emission forms includes control of switching on and off of illuminator 23 and control of the color of light. Illumination controller 34 is, for example, a circuit.

In addition, when for instance the user performs an operation to activate intraoral camera 10 on touch screen 52 of portable terminal 50, a corresponding signal is transmitted from portable terminal 50 to signal processor 30 (illumination controller 34) via communicator 40. Illumination controller 34 of signal processor 30 controls the light emission forms of first to fourth LEDs 23A to 23D in accordance with the received signal.

In addition to the above programs, memory 35 stores, for example, dentition images (image data) captured by imaging unit 21 and various setting data items. The setting data items include a table associating areas with illumination intensities for the respective areas. Memory 35 is embodied as, for example, semiconductor memory such as RAM and ROM. However, memory 35 is not limited to the semiconductor memory. Memory 35 is an example of storage.

In addition, signal processor 30 is further included in handle 10b of intraoral camera 10 and may include a lens driver (not illustrated) and a power supply controller (not illustrated). Here, the lens driver controls an actuator that is a composition adjustment mechanism and an actuator that is a focus adjustment mechanism. The power supply controller distributes the power of a battery. The lens driver and the power supply controller are, for example, circuits.

When for instance the user performs an operation related to composition adjustment or focus adjustment on touch screen 52 of portable terminal 50, a corresponding signal is transmitted from portable terminal 50 to signal processor 30 via communicator 40. In accordance with the received signal, signal processor 30 transmits a control signal to the lens driver in order to perform the composition adjustment or the focus adjustment. In addition, for instance, in accordance with the dentition image received from image processor 32, signal processor 30 may calculate the amount of control for the actuator required for the composition adjustment or the focus adjustment. Then, a control signal corresponding to the calculated amount of control may be transmitted to the lens driver.

Communicator 40 is a wireless communication module that performs wireless communication with portable terminal 50. Communicator 40 is included in, for example, handle 10b of intraoral camera 10 and performs wireless communication with portable terminal 50 in accordance with the control signal from signal processor 30. Communicator 40 performs, with portable terminal 50, wireless communication complying with an existing communication standard, such as Wi-Fi (registered trademark) or Bluetooth (registered trademark). A dentition image showing teeth is transmitted from intraoral camera 10 to portable terminal 50 via communicator 40, and an operation signal is transmitted from portable terminal 50 to intraoral camera 10 via communicator 40.

Here, the plurality of intraoral areas are described with reference to FIGS. 3 and 4. FIG. 3 illustrates teeth inside a mouth. The types of teeth are, for example, the central incisors, the lateral incisors, and the canines illustrated in FIG. 3. The positions of the teeth are indicated by, for example, a maxilla, a mandible, the right side, and the left side.

FIG. 4 illustrates an example of intraoral areas according to Embodiment 1. In FIG. 4, each of the teeth inside the mouth belongs to one of the following six areas: the maxillary left area, the maxillary front area, the maxillary right area, the mandibular left area, the mandibular front area, and the mandibular right area. The six areas are an example of a plurality of areas inside the mouth defined by dividing a dentition into sections. It should be noted that an example in which the entire area is divided into the six areas is described here. However, the number of areas may be any number. For instance, the entire area may be divided into the two areas: a maxillary area and a mandibular area. In addition, each area may further be divided on the basis of imaging directions. For instance, as illustrated in FIG. 4, each area may be divided into a buccal-side area and a lingual-side area on the basis of the two imaging directions. In addition, an example in which each tooth does not belong to more than one area is described here. However, one or more of the teeth may belong to two or more areas. For instance, a tooth near the boundary of two adjacent areas may belong to both areas. For instance, in FIG. 4, the canine at the left end of the maxillary front area, which is the third tooth in the palmer notation system, may belong to both of the maxillary front area and the maxillary left area.

In addition, as illustrated in FIGS. 3 and 4, the plurality of intraoral areas include two or more areas included in the maxillary area (the maxillary left area, the maxillary front area, and the maxillary right area) and two or more areas included in the mandibular area (the mandibular left area, the mandibular front area, and the mandibular right area).

Intraoral camera 10 can control the illumination intensity of illuminator 23 according to the intraoral area whose image is being/is to be captured by imaging unit 21. When for instance the user moves intraoral camera 10 to a given position inside the user's mouth, intraoral camera 10 can detect the intraoral area whose image is to be captured by imaging unit 21 at the given position and illuminate the area at an illumination intensity suitable for the detected area.

A specific example of a method of identifying the area and the imaging direction from the sensing data of the acceleration sensor is described below. Area detector 33 determines whether the area is maxillary or mandibular according to output Az by the acceleration sensor for the z-axis direction. Here, when a maxillary-dentition image is captured, an imaging plane faces upward to no small extent. When a mandibular-dentition image is captured, the imaging plane faces downward to no small extent. Thus, when Az > 0, area detector 33 determines that the area corresponding to the image data is mandibular. When Az ≤ 0, area detector 33 determines that the area corresponding to the image data is maxillary.

Then, a method of identifying which area of the maxilla the area is, when it is determined that the area is maxillary, is described below. Area detector 33 determines whether a tooth in the area is an anterior tooth, according to output Ay by the acceleration sensor for the y-axis direction. Here, when an image of an anterior tooth is captured, intraoral camera 10 is relatively horizontal. However, when an image of a molar is captured, intraoral camera 10 has to be tilted due to interference of lips. Thus, when Ay ≤ threshold a, area detector 33 determines that the area is the maxillary front area. The horizontal state and the tilted state are examples of the orientation.

After determining that the area is the maxillary front area, area detector 33 further determines whether the area is the buccal-side area or the lingual-side area, according to output Ax by the acceleration sensor for the x-axis direction. Here, the orientation of the imaging plane when an image is captured from the buccal side is opposite to the orientation of the imaging plane when an image is captured from the lingual side. Thus, when Ax > 0, area detector 33 determines that the area is the maxillary front buccal-side area. When Ax ≤ 0, area detector 33 determines that the area is the maxillary front lingual-side area.

Meanwhile, when determining that the area is not the maxillary front area, area detector 33 identifies the orientation of the imaging plane according to output Ax by the acceleration sensor for the x-axis direction. Specifically, when Ax > 0, area detector 33 determines that the area is the maxillary right buccal-side area or the maxillary left lingual-side area. When Ax ≤ 0, area detector 33 determines that the area is the maxillary left buccal-side area or the maxillary right lingual-side area.

Area detector 33 further narrows down the areas according to the area identified in the previous processing. Specifically, when area detector 33 determines whether the area is the maxillary right buccal-side area or the maxillary left lingual-side area, if the previously identified area is one of the maxillary front buccal-side area, the maxillary right buccal-side area, the maxillary right lingual-side area, the mandibular front buccal-side aera, the mandibular right buccal-side area, and the mandibular right lingual-side area, area detector 33 estimates that the current area is the maxillary right buccal-side area. If the previously identified area is one of the maxillary front lingual-side area, the maxillary left buccal-side area, the maxillary left lingual-side area, the mandibular front lingual-side area, the mandibular left buccal-side area, and the mandibular left lingual-side area, area detector 33 estimates that the current area is the maxillary left lingual-side area.

When area detector 33 determines whether the area is the maxillary left buccal-side area or the maxillary right lingual-side area, if the previously identified area is one of the maxillary front buccal-side area, the maxillary left buccal-side area, the maxillary left lingual-side area, the mandibular front buccal-side area, the mandibular left buccal-side area, and the mandibular left lingual-side area, area detector 33 estimates that the current area is the maxillary left buccal-side area. If the previously identified area is one of the maxillary front lingual-side area, the maxillary right buccal-side area, the maxillary right lingual-side area, the mandibular front lingual-side area, the mandibular right buccal-side area, and the mandibular right lingual-side area, area detector 33 estimates that the current area is the maxillary right lingual-side area. The estimation is based on a high probability of the imaging plane being moved to keep the amount of the movement of the imaging plane and the orientation change of the imaging plane to a minimum.

In addition, similar determination is performed for the mandible. Specifically, area detector 33 determines whether the tooth in the area is an anterior tooth, according to output Ay by the acceleration sensor for the y-axis direction. Specifically, when Ay ≤ threshold b, area detector 33 determines that the area is the mandibular front area.

After determining that the area is the mandibular front area, area detector 33 further determines whether the area is the buccal-side area or the lingual-side area, according to output Ax by the acceleration sensor for the x-axis direction. Specifically, when Ax < 0, area detector 33 determines that the area is the mandibular front buccal-side area. When Ax ≥ 0, area detector 33 determines that the area is the mandibular front lingual-side area.

Meanwhile, when determining that the area is not the mandibular front area, area detector 33 identifies the orientation of the imaging plane according to output Ax by the acceleration sensor for the x-axis direction. Specifically, when Ax > 0, area detector 33 determines that the area is the mandibular right buccal-side area or the mandibular left lingual-side area. When Ax ≤ 0, area detector 33 determines that the area is the mandibular left buccal-side area or the mandibular right lingual-side area.

When area detector 33 determines whether the area is the mandibular right buccal-side area or the mandibular left lingual-side area, if the previously identified area is one of the mandibular front buccal-side area, the mandibular right buccal-side area, the mandibular right lingual-side area, the mandibular front buccal-side area, the maxillary right buccal-side area, and the maxillary right lingual-side area, area detector 33 estimates that the current area is the mandibular right buccal-side area. If the previously identified area is one of the mandibular front lingual-side area, the mandibular left buccal-side area, the mandibular left lingual-side area, the maxillary front lingual-side area, the maxillary left buccal-side area, and the maxillary left lingual-side area, area detector 33 estimates that the current area is the mandibular left lingual-side area.

When area detector 33 determines whether the area is the mandibular left buccal-side area or the mandibular right lingual-side area, if the previously identified area is one of the mandibular front buccal-side area, the mandibular left buccal-side area, the mandibular left lingual-side area, the maxillary front buccal-side area, the maxillary left buccal-side area, and the maxillary left lingual-side area, area detector 33 estimates that the current area is the mandibular left buccal-side area. If the previously identified area is one of the mandibular front lingual-side area, the mandibular right buccal-side area, the mandibular right lingual-side area, the maxillary front lingual-side area, the maxillary right buccal-side area, and the maxillary right lingual-side area, area detector 33 estimates that the current area is the mandibular right lingual-side area.

In the above processing, one of the maxillary front buccal-side area, the maxillary front lingual-side area, the maxillary right buccal-side area, the maxillary left lingual-side area, the maxillary left buccal-side area, the maxillary right lingual-side area, the mandibular front buccal-side area, the mandibular front lingual-side area, the mandibular right buccal-side area, the mandibular left lingual-side area, the mandibular left buccal-side area, and the mandibular right lingual-side area is identified as the current area.

It should be noted that the above identification algorithm is just an example, and any identification algorithm may be used as long as it is possible to identify the area from output Ax, output Ay, and output Az by the acceleration sensor. For instance, rather than using the values of output Ax, output Ay, and output Az as variables, a secondary variable obtained by appropriately combining output Ax, output Ay, and output Az may be used for the identification. The secondary variable can optionally be set to, for example, Ay/Az, Ax·Ax + Ay-Ay, and Az - Ax. Alternatively, the area may be identified after axis acceleration information items Ax, Ay, and Az are converted into angle information items (orientation angles) α, β, and γ. For instance, the angle of the x-axis relative to the direction of gravitational acceleration may be defined as roll angle α, the angle of the y-axis relative to the direction of gravitational acceleration may be defined as pitch angle β, and the angle of the z-axis relative to the direction of gravitational acceleration may be defined as yaw angle γ. In addition, the threshold used in each identification can be determined from the results of, for example, clinical tests.

In addition, in the above example, the imaging direction is identified from the two imaging directions: the imaging direction when an image is captured from the buccal side and the imaging direction when an image is captured from the lingual side. However, the imaging direction may be identified from three imaging directions including the imaging direction when an image of the top of a tooth is captured. For instance, it is possible to determine whether the imaging direction is the direction when an image of the top of a tooth is captured, on the basis of the state in which the imaging plane is more horizontal when an image of the top of a tooth is captured, compared with when an image is captured from the buccal side and when an image is captured from the lingual side.

In the example described above, the image capturing area of imaging unit 21 and the imaging direction of imaging unit 21 are identified using the three-axis acceleration sensor of sensor 22. However, the image capturing area and the imaging direction may be identified using a three-axis gyro sensor. The three-axis gyro sensor outputs, for example, the amount of angle change caused by movement around the x-axis, the amount of angle change caused by movement around the y-axis, and the amount of angle change caused by movement around the z-axis. That is, for the three-axis gyro sensor, the amount of change for each axis may be added under the condition that the initial states of the x-axis, the y-axis, and the z-axis are set to given states. By doing so, the image capturing area of imaging unit 21 and the orientation of the imaging plane of intraoral camera 10 (the imaging direction) may be identified.

It should be noted that the image capturing area of imaging unit 21 and the orientation of the imaging plane of intraoral camera 10 may be identified using the combination of the three-axis acceleration sensor and the three-axis gyro sensor.

### [1-2. Operation of Intraoral Camera]

An operation of intraoral camera 10 having the above configuration (an illumination control method) is described below with reference to FIG. 5. FIG. 5 is a flowchart illustrating an operation of intraoral camera 10 according to Embodiment 1. An example in which area detector 33 detects, using the sensing data, the area whose image is being/is to be captured by imaging unit 21, is described below.

As illustrated in FIG. 5, image data is generated by the user capturing an image of teeth and gums inside the user's mouth with intraoral camera 10 (S10). The image data is output from imaging unit 21 to image processor 32. Image processor 32 performs the image processing on the image data obtained from imaging unit 21 and then outputs the image data which has undergone the image processing to camera controller 31 and area detector 33. In step S10, for instance, control may be performed so that illuminator 23 illuminates at an illumination intensity registered as an initial setting illumination intensity.

Then, area detector 33 detects the orientation of imaging unit 21 according to the sensing data of sensor 22 obtained when the image was captured in step S10 (S20). For instance, the sensing data may be obtained for each frame of the image data.

Then, area detector 33 detects, from the orientation of imaging unit 21, the area whose image is being captured by imaging unit 21 among a plurality of intraoral areas (S30). For instance, area detector 33 detects the area whose image is being captured by imaging unit 21 among the six areas illustrated in FIG. 4. Area detector 33 may further detect the imaging direction of imaging unit 21. For instance, area detector 33 may detect the image capturing area and the imaging direction, which, for example, indicate that an image of the maxillary front area is being captured from the lingual side and that an image of the mandibular front area is being captured from the front of the user (from outside of the mouth toward the inside of the mouth). In addition, area detector 33 may detect, from the orientation of imaging unit 21, whether the area whose image is being captured by imaging unit 21 is a first area. Area detector 33 outputs information indicating the detected area (a detection result) to illumination controller 34.

Then, illumination controller 34 controls the illumination intensity of illuminator 23 according to the area detected by area detector 33 (S40). Thus, illumination controller 34 can cause illuminator 23 to emit light at the brightness suitable for the area whose image is being captured by imaging unit 21. After illumination controller 34 controls the illumination intensity in step S40, imaging unit 21 captures an image of the image capturing area (teeth). In this way, it is possible to obtain image data having desired brightness.

It should be noted that step S10 need not be performed. In addition, the processing execution timing of steps S20 to S40 is not limited to a particular timing. The processing of steps S20 to S40 may be performed at predetermined time intervals and may be performed every time when a predetermined or more change is seen in at least one of the orientation or position of imaging unit 21.

Here, an example of a combination of the area detected by area detector 33 and the control details of illumination controller 34 is described. First, a case in which the plurality of areas include the first area(s) and the second area(s) is described.

When the plurality of areas include the first area(s) and the second area(s), illumination controller 34 may control illuminator 23 to cause the illumination intensity of illuminator 23 for the first area(s) to be different from that of illuminator 23 for the second area(s). Illumination controller 34 may control illuminator 23 to cause the illumination intensity for the second area(s) to be higher than that for the first area(s).

Then, a case in which the illumination intensity is different depending on the imaging direction of imaging unit 21 is described. Specifically, a case in which the illumination intensity is different depending on from which of the buccal side or the lingual side inside the mouth imaging unit 21 is capturing the image of the area, is described.

In step S30, on the basis of at least one of the image data captured by imaging unit 21 or the orientation of imaging unit 21, area detector 33 further detects from which of the buccal side or the lingual side inside the mouth imaging unit 21 is capturing an image of the area. Area detector 33 outputs, to illumination controller 34, information indicating the area whose image is being captured by imaging unit 21 and information indicating from which of the buccal side or the lingual side imaging unit 21 is capturing an image (e.g., the imaging direction).

Illumination controller 34 sets the illumination intensity suitable for the area whose image is being captured by imaging unit 21 and the imaging direction of imaging unit 21 in accordance with the table associating the image capturing area and the imaging direction with an illumination intensity. Illumination controller 34 controls illuminator 23 so that the illumination intensity is different between when imaging unit 21 is capturing an image of the area from the buccal side and when imaging unit 21 is capturing an image of the area from the lingual side. That is, even if the area is the same, when the imaging direction is different, illumination controller 34 controls illuminator 23 so that the area is illuminated at a different illumination intensity. In this case, in the example illustrated in FIG. 4, 12 combinations can be made from areas whose images are to be captured by imaging unit 21 and the imaging directions of imaging unit 21, and a table associating each of the 12 combinations with an illumination intensity is generated in advance.

For instance, when the current area is the maxillary front area or the mandibular front area (the first area) and an image of the area is being captured from the lingual side, illumination controller 34 controls illuminator 23 so that the area is illuminated at a higher illumination intensity than when the area is being captured from the front of the user. This is an example of first control. In addition, when the current area is one of the maxillary right area, the maxillary left area, the mandibular right area, or the mandibular left area (the second area) and an image of the area is being captured from the lingual side, illumination controller 34 controls illuminator 23 so that the area is illuminated at a lower illumination intensity than when the area is being captured from the buccal side. This is an example of second control.

Thus, illumination controller 34 may perform control so that the illumination intensity of illuminator 23 (the high and low relationship of the illumination intensity) is different between the first control and the second control.

In addition, illumination controller 34 may control illuminator 23 so that the illumination intensity gradually increases from the maxillary front area toward the maxillary right area and toward the maxillary left area and gradually increases from the mandibular front area toward the mandibular right area and toward mandibular left area.

### [Variation of Embodiment 1]

The above-mentioned identification of, for example, the tooth area and the imaging direction (generation of area information) based on the orientation of intraoral camera 10 is performed on the assumption that the user who is, for example, standing upright or sitting in a chair faces forward. Meanwhile, when, for example, a dentist captures an image of patient's teeth, the image may be captured in a state in which a user (patient) lies face upward. In such a case, the relationship between the vertical axis and the teeth differs from that in a state in which the user faces forward. Thus, identification may not be performed properly. Hereinafter, a method that enables proper identification even in such a case is described.

FIG. 6 illustrates relationships between projective planes and standing user BD. Here, the projective planes are virtual planes relative to user BD and include the three planes: frontal plane 110, sagittal plane 111, and transverse plane 112. Frontal plane 110 divides the body of user BD into anterior and posterior halves and is perpendicular to a floor surface. Sagittal plane 111 passes through the body of user BD from front to back and divides the body of user BD into right and left halves. Sagittal plane 111 is perpendicular to the floor surface. Transverse plane 112 is parallel to the floor surface and divides the body of user BD into superior (upper) and inferior (lower) halves. Transverse plane 112 is perpendicular to both frontal plane 110 and sagittal plane 111.

In addition, axes of motion are a vertical axis, a sagittal-transverse axis, and a frontal-transverse axis. The x-axis in FIG. 6 is the frontal-transverse axis. The sagittal-transverse axis is an axis in a left-right (horizontal) direction and the rotational axis of motions such as forward backward bend and flexion and extension on sagittal plane 111. The y-axis in FIG. 6 is the sagittal-transverse axis. The sagittal-transverse axis is an axis in an anteroposterior direction and is the rotational axis of motions such as side bend and abduction and adduction on frontal plane 110. The z-axis in FIG. 6 is the vertical axis. The vertical axis is an axis in a vertical direction and the rotational axis of motion such as rotational motion on transverse plane 112.

FIGS. 7 and 8 each illustrate an example of the posture of user BD during use of intraoral camera 10, according to the variation.

As illustrated in FIG. 7, when user BD standing upright or sitting in a chair uses intraoral camera 10, it is possible to assume that the user is standing. At this time, vertical axis Z0 of the body of user BD (the z-axis) is perpendicular to the floor surface and identical to the direction in which gravitational acceleration acts.

Meanwhile, as illustrated in FIG. 8, when for instance a dentist uses intraoral camera 10 for user BD lying on a dental treatment chair, frontal plane 110 of the upper body of user BD is tilted parallel to the back of the dental treatment chair. That is, since frontal plane 110 of user BD is tilted, vertical axis Z1 of the user whose upper body is tilted parallel to the back of the dental treatment chair tilts relative to vertical axis Z0 of the body of user BD standing upright.

FIG. 9 is a flowchart illustrating image processing according to the variation. FIG. 9 is a flowchart illustrating area detection processing performed by area detector 33 when the posture of user BD changes in this manner. First, area detector 33 obtains and retains the initial orientation of intraoral camera 10 (S110). Specifically, the orientation of intraoral camera 10 when a user operation was performed is obtained as the initial orientation on the basis of a user operation. For instance, the initial orientation is obtained on the basis of a user operation for portable terminal 50. Alternatively, the initial orientation is obtained when, for example, a button provided on intraoral camera 10 is pressed. For instance, orientation information on three axes relative to the vertical direction, obtained by sensor 22 which is a six-axis sensor is obtained as the initial orientation. Portable terminal 50 or intraoral camera 10 retains the initial orientation.

The initial orientation may be a state in which the imaging plane of intraoral camera 10 is parallel to the front surfaces of anterior teeth and the axial direction of intraoral camera 10 and a height direction of the anterior teeth match when viewed in the direction perpendicular to the imaging plane. Here, the axial direction is the direction from handle 10b toward head 10a, passing through the center of intraoral camera 10 in the longitudinal direction of intraoral camera 10. In addition, for instance, the axial direction is the direction passing through the center of imaging plane (image data) in a vertical direction (column direction) of the imaging plane (image data).

It should be noted that a state in which the initial orientation is obtained is not limited to the above example and may be a given state based on at least one tooth. For instance, one or more teeth other than the anterior teeth may be used. In addition, the state in which the axial direction of intraoral camera 10 matches the height (longitudinal) direction of the anterior teeth is described as an example of the initial state. However, the initial state may be a state in which the axial direction of intraoral camera 10 is orthogonal to the height direction of the anterior teeth (a state in which the axial direction of intraoral camera 10 matches a width (lateral) direction of the anterior teeth). It should be noted that portable terminal 50 may instruct the user to achieve the above state, and the state used in the initial orientation may be the orientation of intraoral camera 10 achieved by the user in accordance with the instruction.

In addition, the orientation of intraoral camera 10 in a state in which the posture of the user and the orientation of intraoral camera 10 have a predetermined relationship may be obtained as the initial orientation. In the initial orientation, the imaging plane of the imaging unit is parallel to frontal plane 110 of user BD, and axial direction LB and vertical axis Z1 of user BD match when viewed in the direction perpendicular to the imaging plane.

It should be noted that the state in which the initial orientation is obtained is not limited to the above example. A given orientation in which the posture of user BD can be associated with the orientation of intraoral camera 10 may be used. In addition, the posture of user BD may be defined using one of or two or more of frontal plane 110, sagittal plane 111, transverse plane 112, the vertical axis, the sagittal-transverse axis, or the frontal-transverse axis. For instance, here, the state in which axial direction LB of intraoral camera 10 matches vertical axis Z1 is specified. However, a state in which axial direction LB of intraoral camera 10 is orthogonal to vertical axis Z1 (a state in which axial direction LB matches the frontal-transverse axis) may be used.

Next, capturing of a tooth image described above is performed. Specifically, by using the initial orientation, area detector 33 adjusts the orientation of intraoral camera 10 obtained during the capturing of the tooth image (S120). That is, by using the initial orientation, area detector 33 adjusts the orientation of intraoral camera 10 to be the same as the orientation of intraoral camera 10 when the user faces forward.

Finally, area detector 33 identifies, for example, the tooth area and the imaging direction (generates area information) from the adjusted orientation (S130).

Thus, area detector 33 can improve the accuracy of area detection by adjusting the orientation of intraoral camera 10 according to the posture of the user. Thus, illumination controller 34 can properly control the illumination intensity of illuminator 23 regardless of the posture of the user.

### [Embodiment 2]

An intraoral camera according to Embodiment 2 is described with reference to FIGS. 10 and 11. It should be noted that differences from Embodiment 1 are mainly explained below and explanations for the same or similar parts in Embodiments 1 and 2 are omitted or simplified.

### [2-1. Configuration of Intraoral Camera System]

A configuration of an intraoral camera system according to Embodiment 2 is described below with reference to FIG. 10. FIG. 10 illustrates a schematic configuration of the intraoral camera system according to Embodiment 2. The intraoral camera system according to Embodiment 2 differs from the intraoral camera system according to Embodiment 1 mainly in the following points: hardware 20a does not include a sensor for detecting the orientation of intraoral camera 11 (e.g., a three-axis acceleration sensor and a three-axis gyro sensor), and signal processor 30a controls the color temperature of reflected light from a tooth. It should be noted that the reflected light from the tooth includes direct reflected light from the tooth (a halation area). In addition, the reflected light may include light emitted from illuminator 23 and reflected off the tooth and the external light reflected off the tooth.

As illustrated in FIG. 10, intraoral camera 11 includes hardware 20a, signal processor 30a, and communicator 40.

Hardware 20a includes the elements of hardware 20 according to Embodiment 1 except for sensor 22 (see FIG. 2). It should be noted that hardware 20a may include a sensor other than a sensor for detecting the orientation of intraoral camera 11.

In addition, imaging unit 21 is capable of generating color image data as image data. Imaging unit 21 includes, for example, a color camera. It should be noted that the image data in Embodiment 2 is color image data unless otherwise noted.

Illuminator 23 is capable of controlling at least one of the brightness of light or the color of light. For instance, illuminator 23 may be capable of emitting white light with two different color temperatures. In addition, illuminator 23 emits illumination light for detecting dental plaque.

Signal processor 30a includes determiner 36 instead of area detector 33 of signal processor 30 according to Embodiment 1.

On the basis of the image data captured by imaging unit 21 (an example of first image data), determiner 36 identifies the position of a tooth whose image is being captured by imaging unit 21 (the position inside the mouth or the area inside the mouth (see FIGS. 3 and 4, for example)).

For instance, determiner 36 may identify the position of the tooth whose image is being captured by imaging unit 21 from the shape of the tooth. For instance, determiner 36 may identify the position of the tooth whose image is being captured by imaging unit 21 from the obtained image data and image data that indicates the typical shapes of teeth (an example of second image data). The second image data is obtained in advance and stored in memory 35.

In addition, determiner 36 may identify the position of the tooth whose image is being captured by imaging unit 21 from the obtained image data and intraoral image data that includes the dentition of the user who uses intraoral camera 11 (an example of third image data). The third image data is obtained by capturing the interior of the user's mouth in advance and prestored in memory 35. In this case, intraoral camera 11 has a registration mode for registering the third image data and identification mode for identifying the position of the tooth whose image is being captured by imaging unit 21 by using the registered third image data. It should be noted that memory 35 may store the color temperatures (e.g., catalogue values or actual values) of light-emitting devices (e.g., LEDs) of illuminator 23.

In addition, determiner 36 may determine that an image captured by imaging unit 21 after the user is informed of an area indicated by notification information is an image of the area. Determiner 36 receives information from portable terminal 50 via communicator 40, the information indicating at least one of the information that the notification information has been received or the information that the area indicated by the notification information is being displayed on touch screen 52. Then, determiner 36 may determine that the image captured by imaging unit 21 after the reception of the information is an image of the area.

Illumination controller 34 controls illumination light from illuminator 23 on the basis of image data so that, regardless of the position of a tooth inside the mouth (that is, regardless of the effects of the external light), the color temperature of reflected light from the tooth (an example of a first color temperature) is brought closer to a desired color temperature or is brought within a desired color temperature range. Illumination controller 34 controls at least one of the illumination intensity or the color temperature of illuminator 23 to maintain a set color temperature or a set color temperature range across the entire area inside the mouth.

The desired color temperature and the desired color temperature range may be set according to the color temperature of white light emitted from the light-emitting devices (e.g., first to fourth LEDs 23A to 23D (see FIG. 1)) of illuminator 23. The desired color temperature and the desired color temperature range include a target color temperature set according to the color temperature of illuminator 23 (an example of a second color temperature). The target color temperature may be set according to, for example, the color temperature of the light-emitting devices of illuminator 23. The target color temperature is, for example, a color temperature corresponding to the center wavelength of the light-emitting devices. However, the target color temperature is not limited to the above example.

The desired color temperature is, for example, the target color temperature. In addition, the desired color temperature range is a color temperature range in which the target color temperature is taken as a reference. The desired color temperature range may be, for example, a range of ± at most 300 K from the target color temperature, a range of ± at most 200 K from the target color temperature, and a range of ± at most 100 K from the target color temperature.

For instance, illumination controller 34 may control at least one of the illumination intensity or the color temperature of illuminator 23 to bring the first color temperature of reflected light from a tooth that is based on the image data closer to the target color temperature. For instance, illumination controller 34 may control at least one of the illumination intensity or the color temperature of illuminator 23 to bring the first color temperature within a predetermined range including the target color temperature.

It should be noted that the target color temperature may be set according to the color temperature of a tooth in an image captured first after intraoral camera 11 is put inside the mouth (the color temperature of reflected light). The color temperature of the tooth in the image captured first is a color temperature corresponding to the color temperature of light emitted from the light-emitting devices and an example of the target color temperature based on the second color temperature of illuminator 23. It should be noted that the color temperature of illumination light from illuminator 23 when first capturing an image of a tooth may be set to a preset color temperature. The tooth in the image captured first may be an anterior tooth or a molar. When the tooth in the image captured first is an anterior tooth, illuminator 23 is controlled to bring the color temperature of the molar closer to the color temperature of the anterior tooth. When the tooth in the image captured first is a molar, illuminator 23 is controlled to bring the color temperature of the anterior tooth closer to the color temperature of the molar. It should be noted that the color temperature of the molar is the color temperature of reflected light reflected off the molar and received by imaging unit 21. Other similar expressions also have the same meaning.

In addition, illumination controller 34 may control at least one of the illumination intensity or the color temperature of illuminator 23, using the result of identification (determination) by determiner 36. When for instance an image of a molar area is being captured, illumination controller 34 may decrease the illumination intensity of illumination light, compared with when an image of an anterior tooth area is being captured.

In addition, illumination controller 34 may control the illumination intensity of illuminator 23 according to the color temperature of a glossy area strongly affected by light emitted from illuminator 23 among a plurality of areas (see FIG. 4) inside the mouth defined by diving a dentition into sections. The glossy area is an area strongly affected by reflection of illumination light, the area being a portion of image data (color image data). The glossy area is an area in which each of the pixel values of three subpixels (RGB) in the image data (that is, red pixel value (X), green pixel value (Y), and bule pixel value (Z)) is at least a threshold. The threshold is, for example, 900 in the 10-bit representation. However, the threshold is not limited to the above example. It should be noted that X, Y, and Z are colors approximately corresponding to R, G, and B and are also referred to as reference color stimuli.

Communicator 40 transmits notification information to portable terminal 50 of the user, the notification information notifying the user of intraoral camera 11 of the area whose image is to be captured by imaging unit 21 among the plurality of areas defined by dividing the dentition into the sections.

When receiving the notification information, portable terminal 50 displays the area indicated by the notification information on touch screen 52. Portable terminal 50 may transmit, to intraoral camera 11, information indicating at least one of the information that the notification information has been received or the information that the area indicated by the notification information is being displayed on touch screen 52.

In the registration mode, touch screen 52 displays, to the user, the position of a tooth (or an area) whose image is to be captured. Tooth image data obtained after the information is displayed (for example, after receiving the information indicating that the area is being displayed) may be stored in memory 35 as image data of the tooth positioned at the displayed position. In the registration mode, image data is obtained for each of all the teeth of the user.

### [2-2. Operation of Intraoral Camera]

An operation of intraoral camera 11 having the above configuration (an illumination control method) is described below with reference to FIG. 11. FIG. 11 is a flowchart illustrating an operation of intraoral camera 11 according to Embodiment 2. Each operation illustrated in FIG. 11 is performed for each tooth or for each tooth area. For instance, at least one of the illumination intensity or the color temperature of illuminator 23 is individually controlled for each tooth or for each tooth area.

As illustrated in FIG. 11, imaging unit 21 receives reflected light from a tooth after light is emitted by illuminator 23 and reflected off the tooth (S210). Imaging unit 21 generates image data by receiving the reflected light and outputs the image data to image processor 32.

Then, determiner 36 detects the color temperature of the reflected light from the image data (S220). Determiner 36 detects, from the image data, the color temperature of the reflected light from the tooth. Specifically, determiner 36 obtains the reference color stimuli (X, Y, Z) from the image data and calculates the color temperature of the reflected light from the reference color stimuli (X, Y, Z).

It should be noted that the color temperature calculated in step S220 may be the mean value of the color temperatures of one tooth and may be, for example, the maximum value, the minimum value, a mode value, or a median value.

Then, determiner 36 determines whether the color temperature detected in step S220 is within the predetermined range (for example, within the desired color temperature range) (S230). Determiner 36 may read out the predetermined range from memory 35 or calculate the predetermined range from the color temperatures of the light-emitting devices of illuminator 23 stored in memory 35.

When determining that the color temperature detected by determiner 36 in step S220 is within the predetermined range (Yes in S230), illumination controller 34 will not change the illumination intensity or the color temperature of illuminator 23. When determining that the color temperature detected by determiner 36 in step S220 is not within the predetermined range (No in S230), illumination controller 34 controls at least one of the illumination intensity or the color temperature of illuminator 23 to bring the color temperature of the reflected light from the tooth closer to the target color temperature (S240).

In the case of No in step S230, illumination controller 34 may perform control to increase the proportion of the illumination light of illuminator 23 in the reflected light by, for example, increasing the illumination intensity without changing the color temperature of illuminator 23. In addition, in the case of No in step S230, illumination controller 34 may control the color temperature of the illumination light of illuminator 23 to bring the color temperature of the reflected light closer to the target color temperature. Illumination controller 34 may control the color temperature of the illumination light of illuminator 23 by controlling the amount of current supplied to two light-emitting devices having different color temperatures.

### [Other Embodiments]

The intraoral camera systems according to the embodiments of the present disclosure are described above. However, the present disclosure is not limited to the embodiments.

For instance, in the examples described above, the intraoral camera mainly used to capture an image of a tooth is used. However, the intraoral camera may be an intraoral-care device including a camera. For instance, the intraoral camera may be, for example, a dental washer including a camera.

In addition, the illuminator according to each embodiment may be embodied as one light-emitting part (e.g., one LED). In addition, the illuminator may be ring-shaped, for example.

In addition, the signal processor described in each embodiment may be embodied as a single device (an illumination control device). Such an illumination control device is embodied as a device for controlling the illuminator of an intraoral camera including an imaging unit that generates image data by capturing an image of a tooth inside the mouth of a user and the illuminator that illuminates the tooth.

In addition, the processing units included in the intraoral camera system according to each embodiment are typically embodied as LSIs, which are integrated circuits. The processing units may be made as individual chips, or one or more of the processing units or all of the processing units may be incorporated into one chip.

In addition, circuit integration may be achieved not only as an LSI but also as a dedicated circuit or a general-purpose processor. A field programmable gate array (FPGA), which can be programmed after manufacturing, or a reconfigurable processor in which the connections and settings of circuit cells inside an LSI are reconfigurable may be used.

In addition, in each embodiment, each of the structural elements may be dedicated hardware or may be achieved by executing a software program suitable for the structural element. The structural element may be achieved by a program executer, such as a CPU or a processor, reading and executing a software program stored in a recording medium, such as a hard disk or semiconductor memory.

In addition, an aspect of the present disclosure may be embodied as, for example, an illumination control method performed by the intraoral camera. In addition, another aspect of the present disclosure may be a computer program for causing a computer to execute characteristic steps included in the illumination control method.

In addition, the intraoral camera according to each embodiment may be embodied as a signal device or as a plurality of devices. When the intraoral camera is embodied as a plurality of devices, it does not matter how the structural elements of the intraoral camera are divided into the plurality of devices. For instance, the input interface may be included in the portable terminal. In this case, the input interface may be a touch panel. The communicator receives, from the portable terminal, a control signal corresponding to input accepted by the input interface of the portable terminal. In addition, for instance, at least one of the functional elements of the signal processor of the intraoral camera may be embodied as the portable terminal or a server (e.g., a cloud server) capable of communicating with the portable terminal. When the intraoral camera is embodied as a plurality of devices, a method of communication between the plurality of devices is not limited to a particular method and may be a wireless communication method or a wired communication method. In addition, the method of communication between the plurality of devices may be a combination of wireless communication and wired communication. It should be noted that the cloud server is a server capable of communicating with the portable terminal via, for example, the internet and may provide the portable terminal with an application for using the intraoral camera. For instance, the user downloads the application from the cloud server and installs the application on the portable terminal. In addition, the cloud server may obtain, via the portable terminal, a dentition image captured by the intraoral camera.

In addition, in the example (see FIG. 4) described in each embodiment, the plurality of intraoral areas each include two or more teeth. However, the number of teeth included in each area is not limited to a particular number and may be one. For instance, each of the teeth may form one area. That is, areas equal in number to the teeth may be present.

In addition, a plurality of kinds of memory may store the table described in each embodiment, which associates the plurality of areas with the illumination intensities for the respective areas. For instance, the table is generated for each usage environment of the intraoral camera (such as indoor, outdoor, a dentist, a residence, and the types of the external light), and may be selected as appropriate by the user operating the input interface.

In addition, when the illumination intensity in a space in which to use the intraoral camera (the light intensity of the external light) is less than or equal to a predetermined value, the illumination controller according to each embodiment need not control the illumination intensity of the illuminator according to the area whose image is being captured by imaging unit 21. The illumination intensity in the space may be obtained from, for example, the portable terminal.

In addition, as another example of detection of the orientation by the area detector according to each embodiment, output Ax, output Ay, and output Az for the x-axis, the y-axis, and the z-axis, respectively may be obtained from the acceleration sensor, and output Bx, output By, and output Bz for the x-axis, the y-axis, and the z-axis, respectively may be obtained from a gyro sensor. Output Ax is output by the acceleration sensor for the x-axis direction (an acceleration component in the x-axis direction). Output Ay is output by the acceleration sensor for the y-axis direction (an acceleration component in the y-axis direction). Output Az is output by the acceleration sensor for the z-axis direction (an acceleration component in the z-axis direction). Output Bx is output around the x-axis from the gyro sensor (an angular velocity component around the x-axis). Output By is output around the y-axis from the gyro sensor (an angular velocity component around the y-axis). Output Bz is output around the z-axis from the gyro sensor (an angular velocity component around the z-axis). When the magnitude of compositional vector A (Ax, Ay, Az) is smaller than a predetermined threshold equivalent to gravitational acceleration, the area detector determines that the intraoral camera stays stills, and output Ax, output Ay, and output Az from the acceleration sensor serve as an orientation vector representing the three-dimensional orientation of the intraoral camera. In addition, when the magnitude of compositional vector A (Ax, Ay, Az) is greater than the predetermined threshold, the area detector determines that the intraoral camera is moving. Then, the amount of angular change of the intraoral camera around each axis of the x-axis, the y-axis, and the z-axis since the area detector most recently determined that the intraoral camera stayed still is determined on the basis of output Bx, output By, and output Bz from the gyro sensor. The orientation vector of the intraoral camera is obtained by rotating, by the determined amount of angular change around each axis, compositional vector A (Ax, Ay, Az) obtained when the area detector most recently determined that the intraoral camera stayed still. The orientation vector indicates the orientation of the imaging unit. It should be noted that the orientation detection method performed by the area detector is not limited to the above method and any existing method may be used.

In addition, the functional block configuration illustrated in each block diagram is a mere example. Two or more functional blocks may be incorporated into one functional block. One functional block may be divided into more than one functional block. A part of the function may be transferred from one functional block to another functional block. In addition, the same hardware or software may process the functions of two or more functional blocks having similar functions in parallel or on a time-sharing basis.

In addition, the order in which the steps are performed in each flowchart is provided as an example to specifically explain the present disclosure. The steps may be performed in order different from the stated order. In addition, one or more of the steps may be performed concurrently (in parallel) with another step.

In addition, another aspect of the present disclosure may be a computer program for causing a computer to execute the characteristic steps included in the illumination control method illustrated in one of FIG. 5, FIG. 9, or FIG. 11.

In addition, for instance, the program may be a program to be executed by the computer. In addition, another aspect of the present disclosure may be a non-transitory computer-readable recording medium having recorded thereon the program. For instance, the program stored in the recording medium may be distributed or put into circulation. For instance, by installing the distributed program on a device including another processor and causing the processor of the device to execute the program, it is possible to cause the device to perform the above processing.

The intraoral camera system(s) according to one aspect or aspects are described above on the basis of the embodiments. However, the present disclosure is not limited to the embodiments. Within the scope of the present disclosure, the one aspect or the aspects may include one or more embodiments obtained by making various changes envisioned by those skilled in the art to each embodiment and one or more embodiments obtained by combining structural elements described in the different embodiments.

### [Industrial Applicability]

The present disclosure is applicable to an intraoral camera system.

### [Reference Signs List]

10, 11 intraoral camera
10a head
10b handle
10c neck
20, 20a hardware
21 imaging unit
22 sensor
23 illuminator
23A first LED
23B second LED
23C third LED
23D fourth LED
24 input interface
30, 30a signal processor
31 camera controller
32 image processor
33 area detector (orientation detector)
34 illumination controller
35 memory (storage)
36 determiner
40 communicator
50 portable terminal
52 touch screen
110 frontal plane
111 sagittal plane
112 transverse plane

## Claims

1. An intraoral camera comprising:
an imaging unit that generates image data by capturing an image of a tooth inside a mouth of a user;
an illuminator that illuminates the tooth; and
an illumination controller that controls at least one of an illumination intensity or a color temperature of the illuminator to bring a first color temperature of reflected light from the tooth closer to a target color temperature based on a second color temperature of the illuminator, the first color temperature being based on the image data.

2. The intraoral camera according to claim 1,
wherein the illumination controller controls the at least one of the illumination intensity or the color temperature of the illuminator to bring the first color temperature within a predetermined range including the target color temperature.

3. The intraoral camera according to claim 1 or 2, further comprising:
a determiner that identifies, from first image data captured by the imaging unit, a position of the tooth whose image is being captured by the imaging unit,
wherein the illumination controller controls the at least one of the illumination intensity or the color temperature of the illuminator by further using a result of the identification by the determiner.

4. The intraoral camera according to claim 3,
wherein the determiner identifies the position of the tooth whose image is being captured by the imaging unit from the first image data and second image data that indicates typical shapes of teeth.

5. The intraoral camera according to claim 3, further comprising:
a storage storing third image data that is pre-captured intraoral image data and includes a dentition of the user of the intraoral camera,
wherein the determiner identifies, from the first image data and the third image data, the position of the tooth whose image is being captured by the imaging unit.

6. The intraoral camera according to claim 3, further comprising:
a communicator that transmits, to the user of the intraoral camera, notification information notifying an area whose image is to be captured by the imaging unit among a plurality of areas inside the mouth which are defined by dividing a dentition of the user into sections,
wherein the determiner determines that an image captured by the imaging unit after the user is notified of the area indicated by the notification information is an image of the area.

7. The intraoral camera according to claim 1 or 2,
wherein the target color temperature is set according to a color temperature of a tooth in an image captured first after the intraoral camera is put inside the mouth, and
the illumination controller controls the at least one of the illumination intensity or the color temperature of the illuminator to bring the first color temperature across an entire intraoral area closer to the target color temperature set.

8. The intraoral camera according to claim 1 or 2,
wherein the illumination controller controls the at least one of the illumination intensity or the color temperature of the illuminator according to a color temperature of a glossy area strongly affected by light emitted from the illuminator among a plurality of areas inside the mouth which are defined by dividing a dentition of the user into sections.

9. An illumination control device that controls an illuminator of an intraoral camera including an imaging unit that generates image data by capturing an image of a tooth inside a mouth of a user and the illuminator that illuminates the tooth, the illumination control device comprising:
an obtainer that obtains the image data generated by the imaging unit; and
an illumination controller that controls at least one of an illumination intensity or a color temperature of the illuminator to bring a first color temperature of reflected light from the tooth closer to a target color temperature based on a second color temperature of the illuminator, the first color temperature being based on the image data.

10. An illumination control method comprising:
generating image data by capturing an image of a tooth inside a mouth of a user;
illuminating the tooth; and
controlling at least one of an illumination intensity or a color temperature of an illuminator to bring a first color temperature of reflected light from the tooth closer to a target color temperature based on a second color temperature of the illuminator, the first color temperature being based on the image data.
